# EUROPEAN PATENT SPECIFICATION

(11) **EP 3 104 805 B1**
(45) Date of publication and mention of the grant of the patent: **24.07.2019**
(21) Application number: 15709784.1
(22) Date of filing: 11.02.2015
(51) Int. Cl.: A61C 7/08, A61C 7/14, A61C 7/20, A61C 7/00, A61N 1/05, A61N 1/20

(54) **ORTHODONTIC TOOTH ADJUSTMENT EXPEDITING SYSTEM**
BESCHLEUNIGUNGSSYSTEM FÜR ORTHODONTISCHE ZAHNKORREKTUR
SYSTÈME D'ACCÉLÉRATION DU RÉGLAGE DENTAIRE ORTHODONTIQUE

(30) Priority: 11.02.2014 US 201414177573
(43) Date of publication of application: 21.12.2016
(73) Proprietor: Rapid Orthodontics, Inc., Columbus, Ohio 43209 (US)
(72) Inventor: DAVIDOVITCH, Zeev, Columbus, Ohio 43209 (US); SANFORD, Robert, Hampton, New Jersey 08827 (US); DAVIDOVITCH, Moshe, 6274913 Tel Aviv (IL)
(74) Representative: Graham Watt & Co LLP
(86) International application number: PCT/US2015/015409
(87) International publication number: WO 2015/123292

(56) References cited:
- US-A- 4 519 779
- US-A1- 2014 023 983

## Description

### TECHNICAL FIELD

The invention relates generally to the field of orthodontic tooth movement and in particularly to an orthodontic tooth adjustment expediting system.

### BACKGROUND

Over the past 25 centuries, orthodontists have been engaged in the process of repositioning teeth from faulty arrangements, or "malocclusions," into healthier and more esthetic arrangements. In order to move teeth, three elements are generally required: 1) force, 2) time and 3) space. The mouth responds to a sustained force placed on a tooth by rearranging, or "remodeling," the alveolar bone around the root of the tooth. Particularly, osteoclasts break down bone in the area where the tooth is being moved to in order to create space for the tooth. As a result, the tooth is able to move in the direction of the force applied thereto. Additionally, osteoblasts form new bone tissue in the area where the tooth is being moved from.

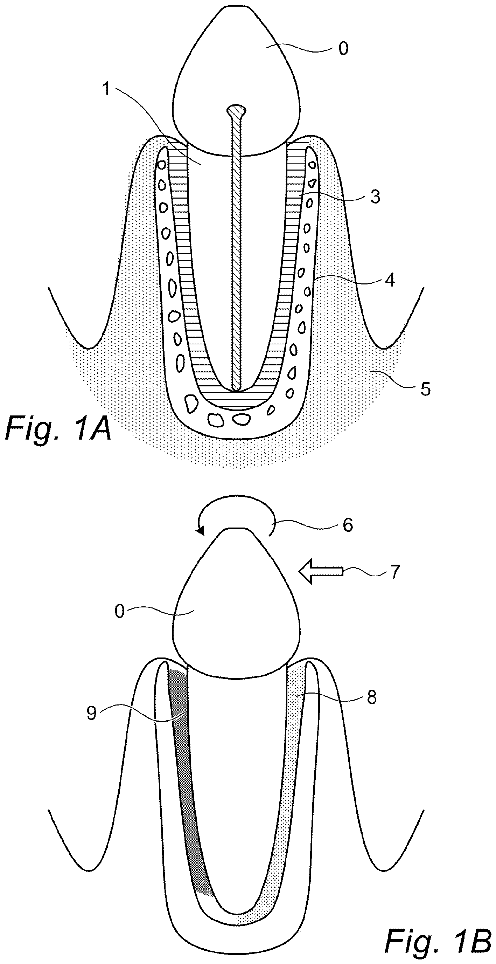

In further detail, FIGs. 1A - 1B illustrate a "remodeling" of the alveolar bone. As illustrated in FIG. 1A, a tooth is comprised of a crown 0 and a root 1. The crown is visible in the mouth, while the root is not, because it is encased in soft connective tissue fibrous mesh known as the periodontal ligament (PDL) 3, and PDL 3 is surrounded by alveolar bone 4. Alveolar bone 4 faces PDL 3 on one side, and is covered by gingival and mucosal tissue (gum) 5 on the other. PDL 3, alveolar bone 4 and gum 5 contain living cells which are responsible for the remodeling of all these tissues in response to orthodontic forces. This remodeling process is the mechanism that facilitates orthodontic tooth movement.

As illustrated in FIG. 1B, physical changes result from application of an orthodontic force to crown 0 of the tooth. The mechanical forces and moments are applied to affect the desired type of tooth movement. Accordingly, a tooth may be extruded, intruded, rotated, tipped, or translated. FIG. 1B illustrates a translatory movement which results from a translational force 7 and a rotation 6 of crown 0 around a center of rotation, or fulcrum, located near the apex of root 1. In order to insure a translatory movement, a force couple is created opposite to rotational force 6. This force couple (generated by the interaction between the bracket and the arch wire for braces, and attachments and aligners for aligner devices) creates a moment that moves root 1 along with crown 0 extending the center of rotation from the apex to infinity. Translational force 7 pushes root 1 against alveolar bone 4, opposite to the point of force application, compressing a portion 9 of PDL 3. Likewise, on the side of the tooth where force 7 is being applied, a portion 8 of PDL 3 is being stretched. Root 1 is displaced within the tooth socket of alveolar bone 4, and the PDL 3 responds by widening portion 8 and compressing portion 9. The compressed portion 9 undergoes removal (resorption), while in the stretched portion 8 new layers of bone are deposited on the surface of the old alveolar bone 4. Over time, alveolar bone 4 remodels, allowing the tooth to assume a new position in the place that translational force 7 caused it to move. This remodeling is a direct result of, and consistent with, the compression/resorption and stretching/deposition activity in PDL 3 and juxtaposed alveolar bone 4. When alveolar bone 4 is pressed upon, as occurs when translational force 7 presses on a tooth and PDL 3 compresses, a short-lived electrical spike can be measured across alveolar bone's 4 matrix. This is known as a piezoelectric effect, characterized by the negative side of the potential being detected on the concave side of the flexed bone and the positive side of the potential being detected on the convex side. In addition, the mechanical stress causes movement of tissue fluids. These fluids contain electrical charges that change the cellular electric polarity, stimulating the cells to remodel their surrounding matrices. These stress-generated streaming potentials (SGP) last about 20 - 30 minutes. When the bone is held in a flexed state, the concave side experiences bone deposition and the convex side experiences bone resorption. As long as the bone remains flexed, this process continues over time until the previously flexed bone appears unflexed or straight.

Over the years, orthodontists have invented devices, generally referred to as "appliances", that allow clinicians to deliver sustained forces to the teeth. Braces, or "orthodontic brackets and arch wires," are the classic appliances that most, if not all orthodontists use. These forces are applied to the crowns of the teeth, then transferred to the dental roots, and from there to the tissues that surround the roots, consisting of the periodontal ligament, a thin soft tissue sleeve that embraces each root, separating it from the surrounding alveolar bone. On the outside, the alveolar bone is covered by the gum. All these tissues must remodel, in order to enable the orthodontically treated teeth to move to new positions in the jaw. Braces consist of small brackets that are glued, or "bonded," to the crowns of teeth, and a wire is then inserted into slots in the brackets and held into place with a ligature or clip. The brackets do not generate forces themselves, but rather transfer forces to the teeth from the deflected wire, when it is inserted into the slot in the bracket and held in place by the ligature. The wire has a "memory," i.e., a characteristic by which the wire tends to return to its original shape, and in doing so, exerts a force on the bracket that is in turn transmitted to the tooth. Through the application of various types, shapes and sizes of wires, the teeth eventually align themselves into the desired position in the dental arch. The technical term used among orthodontists to describe braces is "comprehensive fixed appliance". The tooth movement is clarified by Wolff's Law, which states, in effect, that bone under mechanical stress is remodeled to accommodate and reduce the stress.

Bone cells are responsive to various physical and chemical agents, amongst them: mechanical force and electricity. When direct current, in the order of 20 µA, is applied to bone, deposition of new bone matrix occurs near the cathode, while destruction of old bone is found near the anode. This feature, as well as the ability of bone cells to respond simultaneously to force and electricity, create a favorable environment for acceleration of the rate of bone remodeling and, consequently, the speed of tooth movement. Due to a synergistic relationship between applied force and applied electricity, when both force and electricity are applied simultaneously less of each are necessary to achieve an optimally enhanced osteogenic response from the bone.

Typical orthodontic appliances must be worn by the patient for extended periods of time, often several years or more, in order to achieve the desired results. The classic orthodontic treatment that requires the continuous application of forces to attain the planned tooth movement is expensive, as it requires frequent modifications of the magnitude and direction of forces applied to different teeth, to achieve the necessary progress, requiring frequent adjustments by the treating orthodontist. Moreover, wearing the mechanical fixtures known as "braces" creates a considerable discomfort for the patient, and at the same time this condition will cause an aesthetic concern to the patient as the metallic fixtures (Braces) are visible to other people. In addition, the braces promote the accumulation of bacteria and viruses, harmful to the teeth and their surrounding tissues.

In order to overcome some of the above disadvantages, orthodontic appliances have been developed that can be inserted and removed by the patient, and worn part-time. A myriad of removable appliances have been developed over the years, but the vast majority of them are not "comprehensive" in nature, i.e. the removable appliances address specific movements or malocclusions, and are only used for a certain limited period of time. Treatment with removable appliances is often used in conjunction with braces or other appliances.

U.S. Patent 4,153,060, issued on May 8, 1979 to Korostoff et al., teaches a method and apparatus for electrically stimulating alveolar bone remodeling and tooth movement in the mouths of humans. A positive electrode is placed on the gum surface adjacent the bone structure which is to be resorbed. A negative electrode is placed on the gum surface adjacent the bone tissue which is to be accreted or built up. A current source is connected, such that a small current flows between the electrodes, which have the effect of stimulating bone growth in a specific direction. In a particular arrangement, the electrodes are placed on the gum surface adjacent a tooth, the positive electrode on the side towards which the tooth should move, and the negative on the side from which the tooth will move. Application of a small current to the electrodes will enhance the repositioning of the tooth in conjunction with normal orthodontic practices. However, Korostoff fails to provide a comprehensive and effective system for reducing orthodontic treatment time. Additionally, the electrodes of Korostoff tend to cause excessive irritation of the gums. Although several decades have passed, the method of Korostoff has not achieved wide use by orthodontists.

U.S. Patent 4,854,865, issued August 8, 1989 to Beard et al., teaches an improved method of orthodontic electro-osteogenesis using a biocompatible anode in contact with an electrolytic gel between the anode and epithelial gingiva at an area of osteoclastic or osteoblastic activity, and a biocompatible cathode in contact with a different type of electrolytic gel between the cathode and epithelial gingiva at an area of osteoclastic or osteoblastic activity. Current is then applied across the anode and cathode to stimulate osteogenesis. This method stimulates osteogenesis, which is an important element in tooth movement, but is unable to demonstrate how to achieve desirable results, or to enable to complete orthodontic treatment in a shorter amount of time.

U.S. patent application publication US 2014/0023983, published January 23, 2014 to Lowe et al., is addressed to an electro-orthodontic appliance which helps accelerate orthodontic tooth movement through the application of a controlled electric current to gum and teeth, thus stimulating osteogenesis. Lowe does not provide a comprehensive solution for enhancing orthodontic tooth movement in a plurality of treatment situations, including a combination of bodily and tipping movements of a plurality of teeth. Additionally, the system of Lowe evokes root resorption due to stimulation of cells residing on the surface of the dental root, which is undesirable.

U.S. patent application publication US 2009/0117513, published May 7, 2009 to Nemeh et al., is addressed to a method and apparatus for concurrent treatment of multiple oral diseases and defects while promoting general oral hygiene utilizing direct current electricity applied to the gingival tissues of the mouth. Nemeh does not provide a system or method of utilizing this electricity for improvement of orthodontic treatment.It is therefore an object of the present disclosure to overcome at least part of the disadvantages of the prior art.

### SUMMARY OF THE INVENTION

Accordingly, it is a principal object of the present invention to overcome disadvantages of prior art arrangements of orthodontic tooth movement. According to the invention, an orthodontic tooth adjustment expediting system as defined in claim 1 is provided.

In one embodiment, the direction of each of the generated first type currents, or the direction of each of the generated second type currents, is arranged to expedite bone resorption or deposition caused responsive to an orthodontic force applied to a tooth disposed within the respective tooth socket. In one further embodiment, the system further comprises a first orthodontic appliance arranged to apply the orthodontic force to each of a plurality of teeth disposed within the plurality of tooth sockets. In another embodiment, responsive to the control circuitry and the power supply, a plurality of the first type currents and a plurality of the second type currents are generated.

According to the invention, the system further comprises: a lingual member arranged to fit the contour of the gum over the lingual side of the alveolar bone, the plurality of lingual electrodes disposed on the lingual member; and a buccal member arranged to fit the contour of the gum over the buccal side of the alveolar bone, the plurality of buccal electrodes disposed on the buccal member. In another embodiment, the system further comprises a plurality of electrical paths isolated from each other, the control circuitry in electrical communication with each of the plurality of lingual electrodes, or with each of the plurality of buccal electrodes, via a respective one of the plurality of electrical paths.

In one embodiment, the control circuitry comprises a user input module arranged to receive a current polarity selection user input, wherein responsive to a particular current polarity selection user input received at the user input module, the direction of the generated first type current between a first of the plurality of lingual electrodes and a first of the plurality of buccal electrodes opposes the direction of the generated first type current between a second of the plurality of lingual electrodes and a second of the plurality of buccal electrodes, or the direction of the generated second type current between a pair of adjacent ones of the plurality of lingual electrodes opposes the direction of the generated second type current between a pair of adjacent ones of the plurality of buccal electrodes, associated with the adjacent ones of the plurality of lingual electrodes. In another embodiment, the control circuitry comprises a user input module arranged to receive an electrode selection user input, wherein responsive to a particular electrode selection user input received at the user input module, a first type current is generated between a first of the plurality of lingual electrodes and a first of the plurality of buccal electrodes, and a first type current is not generated between a second of the plurality of lingual electrodes and a second of the plurality of buccal electrodes, or a second type current is generated between a first pair of adjacent ones of the plurality of lingual electrodes and between a first pair of adjacent ones of the plurality of buccal electrodes, and a second type current is not generated between a second pair of adjacent ones of the plurality of lingual electrodes and between a second pair of adjacent ones of the plurality of buccal electrodes.

In one embodiment, a third of the plurality of lingual electrodes is adapted to be juxtaposed with a first side of a particular tooth socket, and a fourth of the plurality of lingual electrodes is adapted to be juxtaposed with a second side of the particular tooth socket, the second side of the tooth socket opposing the first side of the tooth socket, wherein a third of the plurality of buccal electrodes, associated with the third of the plurality of lingual electrodes, is adapted to be juxtaposed with the first side of the particular tooth socket, and a fourth of the plurality of buccal electrodes, associated with the fourth of the plurality of lingual electrodes, is adapted to be juxtaposed with the second side of the particular tooth socket, and wherein the direction of the first type current generated between the third of the plurality of lingual electrodes and the third of the plurality of buccal electrodes opposes the direction of the first type current generated between the fourth of the plurality of lingual electrodes and the fourth of the plurality of buccal electrodes, or the direction of the second type current generated between the third and fourth of the lingual electrodes opposes the direction of the second type current generated between the third and fourth buccal electrodes. In one further embodiment, the system further comprises a second orthodontic appliance arranged to provide a rotation force to a tooth having a root within the particular tooth socket, the rotational orthodontic force arranged to generate rotational motion of the tooth, the generated rotation motion defining: a first motion vector in a first direction at a portion of a first side of the tooth; and a second motion vector, in a second direction opposing the first direction, at a portion of a second side of the tooth, the second side of the tooth opposing the first side of the tooth; wherein a first axis extending between the third of the plurality of lingual electrodes and the third of the plurality of buccal electrodes is parallel and aligned with the first motion vector, or a second axis extending between the third and fourth of the plurality of lingual electrodes is parallel and aligned with the first motion vector, wherein a third axis extending between the fourth of the plurality of lingual electrodes and the fourth of the plurality of buccal electrodes is parallel and aligned with the second motion vector, or a fourth axis extending between the third and fourth of the plurality of buccal electrodes is parallel and aligned with the second motion vector, and wherein the direction of the first type current generated between the third of the plurality of lingual electrodes and the third of the plurality of buccal electrodes and the direction of the first type current generated between the fourth of the plurality of lingual electrodes and the fourth of the plurality of buccal electrodes are arranged to expedite bone resorption and deposition caused responsive to the provided rotational orthodontic force, or the direction of the second type current generated between the third and fourth lingual electrodes and the direction of the second type current generated between the third and fourth buccal electrodes are arranged to expedite bone resorption and deposition caused responsive to the provided rotation orthodontic force.

In another embodiment, for each of the generated first or second type currents, the control circuitry is arranged such that the generated first or second type current is alternately provided for a predetermined active duration and not provided for a predetermined quiescent duration, the predetermined active duration being 3 - 5 hours.

In one embodiment, the system further comprises a plurality of adjustable pressure elements, each of the adjustable pressure elements arranged to apply an adjustable amount of pressure to a respective one of the plurality of lingual electrodes and the plurality of buccal electrodes. In one further embodiment, each of the adjustable pressure elements is arranged to expand responsive to air being injected therewithin, the adjustable amount of pressure applied responsive to the injected air. In another further embodiment, each of the adjustable pressure elements is arranged to expand responsive to a pressure member being inserted therewithin, the adjustable amount of pressure applied responsive to the inserted pressure member.

In one embodiment, the system further comprises: a temperature sensor arranged to sense the temperature of the gums of the alveolar bone; and a heating element arranged to heat the gums of the alveolar bone, responsive to the sensed temperature. In another embodiment, the system further comprises a pH sensor arranged to sense the pH level of the gums of the alveolar bone, wherein the magnitude of the first type currents or the second type currents are adjusted responsive to the sense pH level.

In one example, a method of expediting orthodontic tooth adjustment is provided, the method comprising: generating a first type current between each of a plurality of lingual electrodes and a particular one of a plurality of buccal electrodes, or a second type current between each of adjacent ones of the plurality of lingual electrodes and between adjacent ones of a plurality of buccal electrodes, each of the plurality of lingual electrodes adapted to be juxtaposed with a lingual side of an alveolar bone and associated with a particular one of a plurality of tooth sockets within the alveolar bone and each of the plurality of buccal electrodes adapted to be juxtaposed with a buccal side of the alveolar bone and associated with a particular one of the plurality of tooth sockets within the alveolar bone, wherein each of the generated first type currents, or each of the generated second type currents, is controlled separately responsive to a control circuitry.

In one embodiment, the direction of each of the generated first type currents, or the direction of each of the generated second type currents, is arranged to expedite bone resorption or deposition caused responsive to an orthodontic force applied to a tooth disposed within the respective tooth socket. In one further example, the method further comprises generating the orthodontic force. In another embodiment, a plurality of the first type currents and a plurality of the second type currents are generated.

In one embodiment, the separate controlling by the control circuitry is via a plurality of electrical paths isolated from each other. In another example, the method further comprises receiving a particular current polarity selection user input, wherein responsive to the received particular current polarity selection user, the direction of the generated first type current between a first of the plurality of lingual electrodes and a first of the plurality of buccal electrodes opposes the direction of the generated first type current between a second of the plurality of lingual electrodes and a second of the plurality of buccal electrodes, or the direction of the generated second type current between adjacent ones of the plurality of lingual electrodes opposes the direction of the generated second type current between adjacent ones of the plurality of buccal electrodes, associated with the adjacent ones of the plurality of lingual electrodes.

In one example, the method further comprises receiving a particular electrode selection user input, wherein responsive to the received particular electrode selection user input, a first type current is generated between a first of the plurality of lingual electrodes and a first of the plurality of buccal electrodes, and a first type current is not generated between a second of the plurality of lingual electrodes and a second of the plurality of buccal electrodes, or a second type current is generated between a first pair of adjacent ones of the plurality of lingual electrodes and between a first pair of adjacent ones of the plurality of buccal electrodes, and a second type current is not generated between a second pair of adjacent ones of the plurality of lingual electrodes and between a second pair of adjacent ones of the plurality of buccal electrodes. In another embodiment, a first of the generated first type currents is associated with a first side of a particular one of the plurality of tooth sockets and a second of the generated first type currents is associated with a second side of the particular one of the plurality of tooth sockets, opposing the first side of the particular one of the plurality of tooth sockets, the direction of the second of the generated first type currents opposing the direction of the first of the generated first type currents, or a first of the generated second type currents is associated with a third side of the particular one of the plurality of tooth sockets and a second of the generated second type currents is associated with a fourth side of the particular one of the plurality of tooth sockets, opposing the third side of the particular one of the plurality of tooth sockets, the direction of the second of the generated second type currents opposing the direction of the first of the generated second type currents.

In one further example, the method further comprises providing a rotational orthodontic force to a tooth having a root within the particular tooth socket, the rotational orthodontic force arranged to generate rotational motion of the tooth, the generated rotation motion defining: a first motion vector in a first direction at a portion of a first side of the tooth, the first of the generated first type currents, or the first of the generated second type currents, parallel and aligned with the first motion vector; and a second motion vector, in a second direction opposing the first direction, at a portion of a second side of the tooth, the second side of the tooth opposing the first side of the tooth, the second of the generated first type currents, or the second of the generated second type currents, parallel and aligned with the second motion vector.

In one embodiment, wherein each of the generated first or second type currents is alternately provided for a predetermined active duration and not provided for a predetermined quiescent duration, the predetermined active duration being 3 - 5 hours. In another example, the method further comprises applying an adjustable amount of pressure to a set of the plurality of lingual electrodes and the plurality of buccal electrodes.

In one further example, the method further comprises injecting air within each of a plurality of adjustable pressure elements, the adjustable amount of pressure applied responsive to the injected air. In another further example, the method further comprises inserting a pressure member within each of a plurality of adjustable pressure elements, the adjustable amount of pressure applied responsive to the inserted pressure members.

In one example, the method further comprises: sensing the temperature of the gums of the alveolar bone; and adjusting the temperature of the gums responsive to the sensed temperature. In another example, the method further comprises: sensing the pH level of the gums of the alveolar bone; and adjusting the magnitude of the generated first type current or second type current responsive to the sensed pH level.

Additional features and advantages of the invention will become apparent from the following drawings and description.

### BRIEF DESCRIPTION OF THE DRAWINGS

For a better understanding of the invention and to show how the same may be carried into effect, reference will now be made, purely by way of example, to the accompanying drawings in which like numerals designate corresponding elements or sections throughout.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:
FIGs. 1A - 1B illustrate bone "remodeling" of an alveolar bone responsive to orthodontic appliance pressure applied to a tooth;
FIGs. 2A - 2D illustrate various high level views of a first embodiment of an orthodontic tooth adjustment expediting system, according to certain embodiments;
FIGs. 3A - 3D illustrate various high level views of a second embodiment of an orthodontic tooth adjustment expediting system, according to certain embodiments;
FIG. 4 illustrates a high level perspective view of a third embodiment of an orthodontic tooth adjustment expediting system, according to certain embodiments;
FIG. 5 illustrates a perspective view of a fourth embodiment of an orthodontic tooth adjustment expediting system; and
FIGs. 6A - 6B illustrate a high level flow chart of a method of expediting orthodontic tooth adjustment.

### DETAILED DESCRIPTION OF THE PREFERRED EMBODIMENTS

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

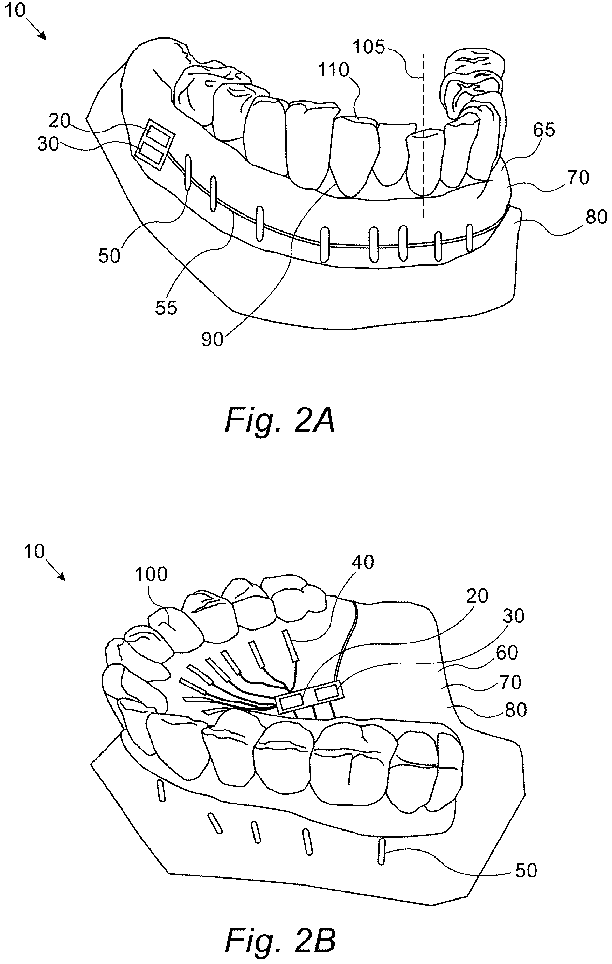

FIGs. 2A - 2B illustrate various a high level perspective views of an orthodontic tooth adjustment expediting system 10, according to certain embodiments. Orthodontic tooth adjustment expediting system 10 comprises: a control circuitry 20; a power supply 30; a plurality of lingual electrodes 40; and a plurality of buccal electrodes 50. In one embodiment, power supply 30 is rechargeable. In one further embodiment, power supply 30 comprises a Nickel-Cadmium rechargeable battery. In another further embodiment, power supply 30 comprises a Lithium rechargeable battery.

Each lingual electrode 40 is adapted to be juxtaposed with a lingual side 60 of an alveolar bone 70. In one embodiment, as will be described below, plurality of lingual electrodes 40 are disposed on a lingual member (not shown) arranged to fit the contour of the gum 80 over lingual side 60 of alveolar bone 70. Each lingual electrode 40 is associated with a particular one of a plurality of tooth sockets 90 within alveolar bone 70. Particularly, in one illustrated embodiment each lingual electrode 40 is positioned between two adjacent tooth sockets 90, i.e. between two adjacent teeth 100. In another embodiment (not shown), a pair of lingual electrodes 40 are positioned between each pair of adjacent tooth sockets 90, a first of the pair of lingual electrodes 40 in proximity to a first of the pair of adjacent tooth sockets 90 and a second of the pair of lingual electrodes 40 in proximity to a second of the pair of adjacent tooth sockets 90.

Each buccal electrode 50 is adapted to be juxtaposed with a buccal side 65 of alveolar bone 70. In one embodiment, as will be described below, plurality of buccal electrodes 50 are disposed on a buccal member (not shown) arranged to fit the contour of the gum 80 over buccal side 65 of alveolar bone 70. Each buccal electrode 50 is associated with a particular one of tooth sockets 90. Particularly, in one illustrated embodiment each buccal electrode 50 is positioned between two adjacent tooth sockets 90, i.e. between two adjacent teeth 100. In another embodiment (not shown), a pair of buccal electrodes 50 are positioned between each pair of adjacent tooth sockets 90, a first of the pair of buccal electrodes 50 in proximity to a first of the pair of adjacent tooth sockets 90 and a second of the pair of buccal electrodes 50 in proximity to a second of the pair of adjacent tooth sockets 90.

In one embodiment, each lingual electrode 40 and buccal electrode 50 is generally longitudinally shaped and extends along an axis generally parallel with a longitudinal axis 105 of each tooth 100. In another embodiment, each lingual electrode 40 and buccal electrode 50 extends from about 1 millimeter apical to the gingival margin to the mucogingival junction of gums 80, further optionally extending 1 - 2 millimeters over the mucosal tissue. In one further embodiment, each lingual electrode 40 and buccal electrode 50 is 5 - 6 millimeters in length.

Power source 30 and control circuitry 20 are coupled to each of plurality of lingual electrodes 40 and plurality of buccal electrodes 50 via a respective one of a plurality of electrical paths 55. Preferably, electrical paths 35 are isolated from each other such that control circuitry 20 is arranged to separately control each lingual electrode 40 and buccal electrode 50, as will be described below. In one embodiment, electrical paths 35 are bundled together within a single isolation material. In one embodiment, each lingual electrode 40 and buccal electrode 50 is arranged to be alternately coupled to a first and a second voltage terminal of power source 30. In such an embodiment, control circuitry 20 is arranged to select for each lingual electrode 40 and buccal electrode 50 to which voltage terminal of power source 30 to be coupled to, as will be described below. In another embodiment, lingual electrodes 40 are each coupled to a first voltage terminal of power source 30 and buccal electrodes 50 are each arranged to be alternately coupled to a second and a third voltage terminal of power source 30, the potential at the second voltage terminal greater than the potential at the first voltage terminal and the potential at the third voltage terminal less than the potential at the first voltage terminal. In such an embodiment, control circuitry 20 is arranged to select for each buccal electrode 50 to which voltage terminal of power source 30 to be coupled to, as will be described below.

In another alternate embodiment, buccal electrodes 50 are each coupled to a first voltage terminal of power source 30 and lingual electrodes 40 are each arranged to be alternately coupled to a second and a third voltage terminal of power source 30, the potential at the second voltage terminal greater than the potential at the first voltage terminal and the potential at the third voltage terminal less than the potential at the first voltage terminal. In such an embodiment, control circuitry 20 is arranged to select for each lingual electrode 40 to which voltage terminal of power source 30 to be coupled to, as will be described below.

Electrical paths 35 connecting control circuitry 20 and power source 30 to buccal electrodes 50 are not shown in FIG. 2B for simplicity.

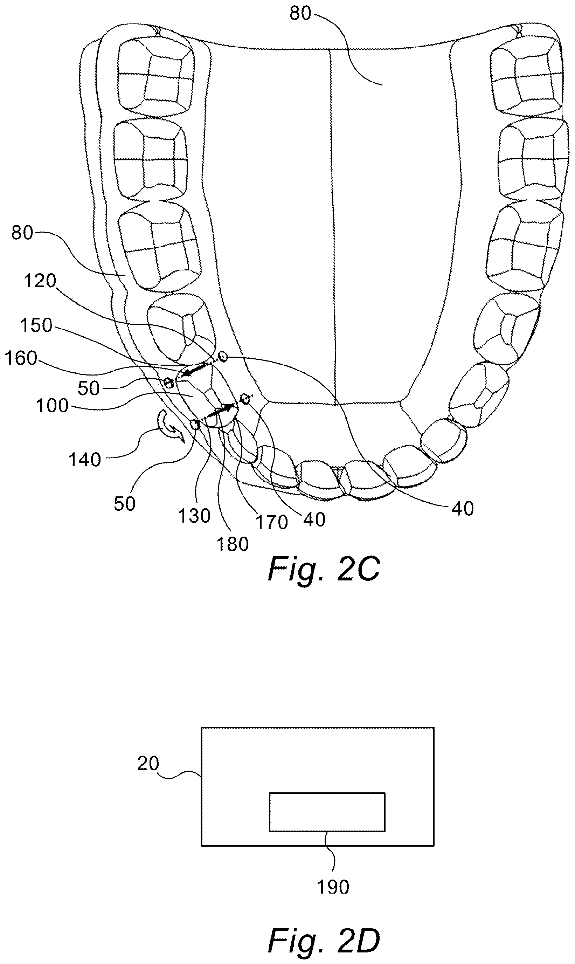

In operation, an orthodontic appliance, such as braces, is connected to teeth 100 and applies pressure to straighten teeth 100. Each tooth 100 which is not in a correct position, i.e. either crooked or out of line with the remainder of teeth 100, has pressure applied thereto by the orthodontic appliance. The pressure applied by the orthodontic appliance moves each of the respective teeth 100 into the appropriate position, as described above in relation to FIGs. 1A - 1B. Control circuitry 20 is arranged to control power source 30 to generate a current between each lingual electrode 40 and an associated lingual electrode 40 or buccal electrode 50. Particularly, each tooth 100 has associated therewith two lingual electrodes 40 and two buccal electrodes 50, as illustrated in FIG. 2C, an axis 120 passing through a first lingual electrode 40 and a first buccal electrode 50 at a first side of tooth 100 and an axis 130 passing through a second lingual electrode 40 and a second buccal electrode 50 at a second side of tooth 100. Only a single pair of lingual electrodes 40 and buccal electrodes 50 are illustrated in FIG. 2C for simplicity, however this is not meant to be limiting in any way. Additionally, the movement of tooth 100 in FIG. 1C is illustrated as a rotational movement, however this is not meant to be limiting in any way.

In the event that tooth 100 needs to be moved lingually, i.e. towards the tongue, a first and a second orthogonal current is generated. The term 'orthogonal current' as used herein means that the current flows in a direction generally orthogonal to the gum line of alveolar bone 70. Particularly, the first orthogonal current is arranged to flow from the first lingual electrode 40 to the first buccal electrode 50, along axis 120, and the second orthogonal current is arranged to flow from the second lingual electrode 40 to the second buccal electrode 50, along axis 130. Responsive to the forces applied by the orthodontic appliance, bone resorption begins on the lingual side of the respective tooth 100 and bone deposition begins on the buccal side of tooth 100. The first and second generated orthogonal currents aid and expedite bone resorption in the vicinity of each of first lingual electrode 40 and second lingual electrode 40, i.e. at the lingual side of tooth 100, and further aid and expedite bone deposition in the vicinity of each of first buccal electrode 50 and second buccal electrode 50, i.e. at the buccal side of tooth 100. As a result, the bone resorption and deposition caused by the orthodontic appliance is enhanced and expedited by the bone resorption and deposition caused by the generated first and second orthogonal currents, thereby expediting the movement of tooth 100.

In the event that tooth 100 needs to be moved buccally, i.e. away from the tongue, a third and a fourth orthogonal current are generated. Particularly, the third orthogonal current is arranged to flow from the first buccal electrode 50 to the first lingual electrode 40, along axis 120. The second orthogonal current is arranged to flow from the second buccal electrode 50 to the second lingual electrode 40, along axis 130. As described above in relation to the generated first and second orthogonal currents, the generated third and fourth orthogonal currents aid and expedite the bone resorption on the lingual side of tooth 100 and the bone deposition of the buccal side of tooth 100.

In the event that tooth 100 needs to be moved along the gum line of alveolar bone 70, a first and second parallel current is generated. The term 'parallel current' as used herein means that the current flows in a direction generally parallel to the gum line of alveolar bone 70 in the vicinity of tooth 100. The first parallel current is arranged to flow from the first lingual electrode 40 to the second lingual electrode 40 and the second parallel current is arranged to flow from the first buccal electrode 50 to the second buccal electrode 50. As described above in relation to the generated orthogonal currents, the generated first and second parallel currents aid and expedite the bone resorption on the side of tooth 100 in the direction that tooth 100 is being moved to and the bone deposition on the side of tooth 100 where tooth 100 is being moved from.

In the event that tooth 100 needs to be rotated, in one embodiment opposing orthogonal currents are generated. Particularly, an orthogonal current flowing in a lingual direction, i.e. towards the tongue, is arranged to flow from the first buccal electrode 50 to the first lingual electrode 40. Additionally, an orthogonal current flowing in a buccal direction, i.e. away from the tongue, is arranged to flow from the second lingual electrode 40 to the second buccal electrode 50. As illustrated in FIG. 2C, a rotation of tooth 100 in a rotational direction 140 can be viewed as movement of a first side 150 of tooth 100 in a buccal direction 160 and movement of a second side 170 of tooth 100, opposing first side 150, in a lingual direction 180. As illustrated, axis 120 extends through first lingual electrode 40 and first buccal electrode 50 and axis 130 extends through second lingual electrode 40 and second buccal electrode 50. Therefore, the orthogonal current flowing from first buccal electrode 50 to first lingual electrode 40 aids and expedites the bone resorption at the buccal end of first side 150 of tooth 100 and the bone deposition at the lingual end of first side 150 of tooth 100. Additionally, the orthogonal current flowing from second lingual electrode 40 to second buccal electrode 50 aids and expedites the bone resorption at the lingual end of second side 170 of tooth 100 and the bone deposition at the buccal end of second side 170 of tooth 100. In another embodiment, opposing parallel current are generate each between the respective pair of lingual electrodes 40 and buccal electrodes 50. In one embodiment, the magnitude of each the above described orthogonal and parallel current is about 20 micro-amperes.

Providing a lingual electrode 40 and a buccal electrode 50 on each side of tooth 100 thus allows for resorption and deposition simultaneously on both sides of tooth 100. In the event that tooth 100 needs to be tipped, i.e. rotated about any rotation axis extending through the middle of the root of tooth 100, the crown of tooth 100 moves in a first direction while the bottom of the root of tooth 100 moves in an opposing direction. As a result, bone resorption and deposition occurs simultaneously on both sides of the center of rotation. The arrangement of orthodontic tooth adjustment expediting system 10 thus allows for enhancement of tipping motion of a tooth 100.

Thus, each tooth 100 can be separately treated responsive to the orthodontic appliance, the movement of each tooth 100 expedited responsive to the respectively generated orthogonal and/or parallel currents. Advantageously, orthodontic tooth adjustment expediting system 10 provides a comprehensive system for expediting orthodontic tooth adjustment in any of a plurality of treatment situations, including a combination of bodily and tipping movement of a plurality of teeth 100. Particularly, each of the generated orthogonal currents and parallel current are separately controlled by control circuitry 20. As a result, for each lingual electrode 40 and buccal electrode 50, control circuitry 20 will control whether a parallel current or an orthogonal current will be generated thereat and whether the particular electrode will act as an anode, i.e. outputting the current, or as a cathode, i.e. receiving the current.

As illustrated in FIG. 2D, control circuitry 20 optionally comprises a user input module 190. User input module 190 is arranged to receive user input, optionally via a connection to an external computer. In one embodiment, user input module 190 is arranged to receive a current polarity selection user input. The directions of the above described generated orthogonal and parallel currents are responsive to the received current polarity selection user input. In another embodiment, user input module 190 is arranged to receive an electrode user input. Control circuitry 20 is arranged to select which lingual electrodes 40 and which buccal electrodes 50 are to be activated for generating and receiving current responsive to the received electrode user input. Particularly, if only several teeth 100 need adjusting, only electrodes associated with those teeth 100 are utilized. In one embodiment, the magnitude of the generated currents can be adjusted via user input module 190.

In one embodiment, control circuitry 20 is arranged to generate the respective orthogonal and parallel currents for a predetermined active duration. Following the predetermined active duration, control circuitry 20 is arranged to not generate the respective orthogonal and parallel currents for a predetermined quiescent duration. Optionally, the predetermined active duration is 3 - 5 hours and the predetermined quiescent duration is 19 - 21 hours. Advantageously, generating the respective orthogonal and parallel currents for only a fraction of the day avoids damage to gums 80 from too much electrical stimulation. Clinical research has shown that an active duration of up to 5 hours a day does not cause irritation of gums 80, while an active duration of less than 3 hours a day does not effectively enhance orthodontic tooth movement. In one embodiment, the predetermined active duration and/or the predetermined quiescent duration can be adjusted via user input module 190.

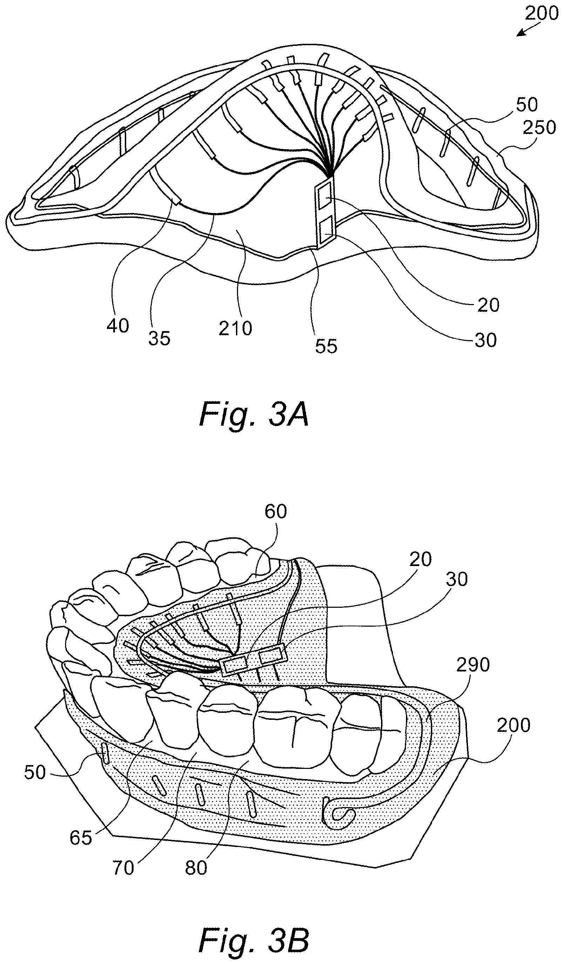

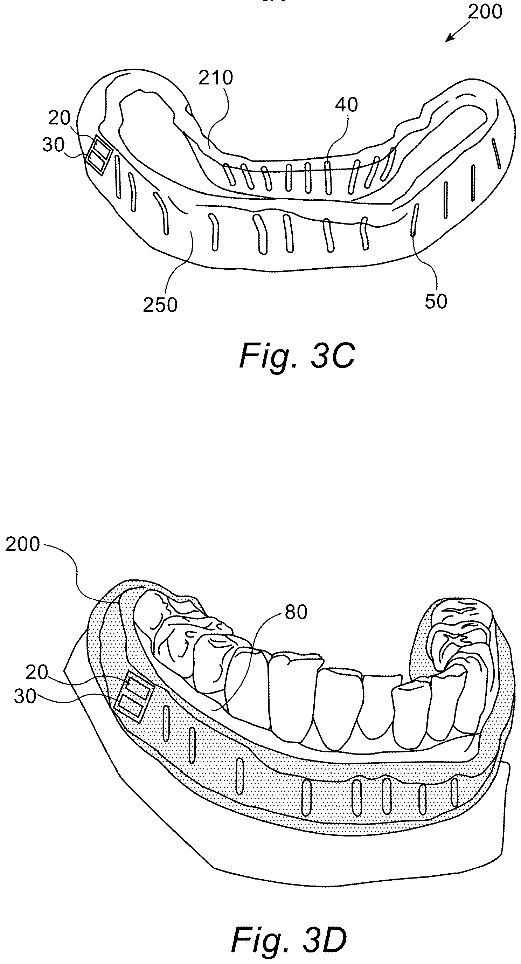

FIGs. 3A - 3D illustrate various high level perspective views of an orthodontic tooth adjustment expediting system 200. Orthodontic tooth adjustment expediting system 200 is in all respects similar to orthodontic tooth adjustment expediting system 10, described above, with the exception that orthodontic tooth adjustment expediting system 200 further comprises: a lingual member 210; and a buccal member 250. In one embodiment, lingual member 210 and buccal member 250 are a single member. In another embodiment, lingual member 210 and buccal member 250 are each comprised of plastic. In a first orthodontic tooth adjustment expediting system 200, illustrated in FIGs. 3A - 3B, lingual member 210 and buccal member 250 are arranged to fit the upper portion of the mouth. Optionally, first orthodontic tooth adjustment expediting system 200 further comprises a structural support member 290 arranged to provide structural support for lingual member 210. In a second orthodontic tooth adjustment expediting system 200, illustrated in FIGs. 3C - 3D, lingual member 210 and buccal member 250 are arranged to fit the lower portion of the mouth.

The plurality of lingual electrodes 40 are disposed on lingual member 210 and the plurality of buccal electrodes 50 are disposed on buccal member 250. Lingual member 210 is arranged to fit the contour of gum 80 over lingual side 60 of alveolar bone 70 such that lingual electrodes 40 are positioned between teeth 100 and buccal member 250 is arranged to fit the contour of gum 80 over buccal side 65 of alveolar bone 70 such that buccal electrodes 50 are positioned between teeth 100, as described above in relation to orthodontic tooth adjustment expediting system 10. Control circuitry 20, power source 30 and electrical paths 35 are disposed on lingual member 210 and buccal member 250. Optionally, power source 30 and electrical paths 35 are sealed within cavities, each between two layers of laminated plastic (not shown), thus being isolated from the oral environment. Advantageously, the isolation from the oral environment keeps the electronics of orthodontic tooth adjustment expediting system 200 protected from the oral environment and keeps the oral environment protected from any toxicity of the electronics of orthodontic tooth adjustment expediting system 200. In one embodiment, the cavities each comprise a vacuum.

As described above, in one embodiment power supply 30 is rechargeable. In such an embodiment, orthodontic tooth adjustment expediting system 200 is arranged to fit on a rechargeable power source. Optionally, power supply power 30 is arranged to be charged wirelessly via an inductive coil. Additionally, in one embodiment, during orthodontic treatment orthodontic tooth adjustment expediting system 200 is arranged to be placed on a base plate (not shown) and power supply 30 is arranged to inductively receive power from the base plate. As a result, the power stored in power supply 30 won't be wasted during the orthodontic treatment which may utilize increased amounts of power to adjust treatment parameters via control circuitry 20 and/or to detect appropriate conductivity between associated ones of lingual electrodes 40 and buccal electrodes 50, as will be described below in relation to orthodontic tooth adjustment expediting system 300.

Preferably, orthodontic tooth adjustment expediting system 200 is arranged to operate in cooperation with any standard orthodontic appliance, including: fixed appliances, such as lingual or buccal braces; and removal aligners, such as the Invisalign clear aligner commercially available from Align Technology Inc. of San Jose, California.

In one embodiment, orthodontic tooth adjustment expediting system 200 can further be utilized to expedite bone filling of tooth extraction sites. Particularly, when a tooth is extracted and no implant is provided for replacing the extracted tooth, the extraction site is left empty. Preferably, bone growth is desired to fill in the empty extraction site. The currents generated by lingual electrodes 40 and buccal electrodes 50 will aid in the deposition of bone in the empty extraction site. Particularly, parallel currents are received by each of the lingual electrodes 40 and buccal electrodes 50 associated with the tooth extraction site, the parallel currents received from adjacent lingual electrodes 40 and buccal electrodes 50. As described above, bone deposition occurs at an electrode which receives current. In another embodiment, where an implant is inserted, the current generated by lingual electrodes 40 and buccal electrodes 50 will aid in the formation of bone around the implant. In one embodiment, alveolar bone loss caused by dentures can be reversed responsive to bone deposition caused by generated parallel currents, as described above. In another embodiment, periodontal bone defects can be repaired responsive to bone deposition caused by generated parallel currents, as described above.

Orthodontic tooth adjustment expediting system 200 thus advantageously provides a comprehensive device, adaptive for a plurality of orthodontic patients. Particularly, as described above, a plurality of parallel and/or orthogonal current can be generated, as required for the particular patient's needs. Additionally, as described above, the currents can be adjusted, and/or new currents may be generated in accordance with the orthodontic treatment process.

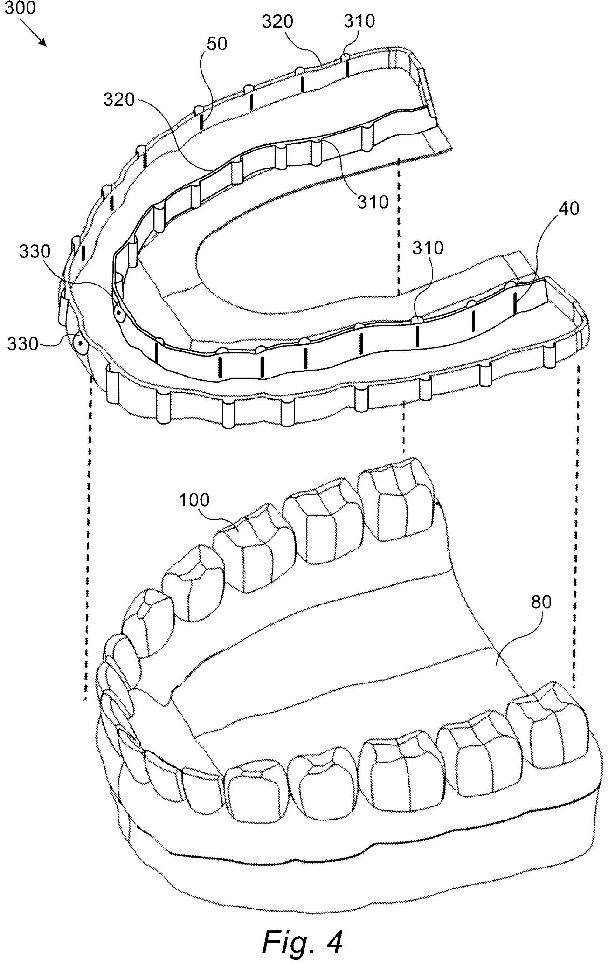

FIG. 4 illustrates a high level perspective open view of an orthodontic tooth adjustment expediting system 300. Orthodontic tooth adjustment expediting system 300 is in all respects similar to orthodontic tooth adjustment expediting system 200 with the exception that orthodontic tooth adjustment expediting system 300 further comprises a plurality of adjustable pressure elements 310. Each adjustable pressure element 310 is juxtaposed with a respective one of lingual electrodes 40 and buccal electrodes 50. In one embodiment, each adjustable pressure element 310 comprises a flexible tubule. During the treatment process, as teeth 100 begin to move into the desired positions, the contact between lingual electrodes 40 and gum 80, and the contact between buccal electrodes 50 and gum 80 may become less optimal due to the movement of teeth 100. In one embodiment, air is pumped into each adjustable pressure element 310 thereby expanding the flexible tubule and applying pressure to the respective lingual electrodes 40 and buccal electrodes 50. Responsive to the applied pressure, the respective lingual electrodes 40 and buccal electrodes 50 are pushed against gum 80, thereby improving the contact therewith. Optionally, as illustrated, adjustable pressure elements 310 are attached to a common air channel 320, air being added to adjustable pressure elements 310 via common air channel 320. Alternatively, a particular gas may be used instead of air. Optionally, common air channel 320 exhibits a unidirectional opening 330, air being injected into common air channel 320 via unidirectional opening 330. In another embodiment, a solid pressure member is inserted into each adjustable pressure element 310, the flexible tubule thereof expanding responsive to the inserted pressure member and applying pressure to the respective lingual electrodes 40 and buccal electrodes 50. Optionally, a plurality of pressure members are provided, with a range of diameters, each thus providing a different amount of pressure to the respective one of lingual electrodes 40 and buccal electrodes 50.

In the embodiment where the pressure is adjusted by air, adjustable pressure elements 310 and common air channels 320 are enclosed between two layers of laminated plastic (not shown) to keep air from escaping orthodontic tooth adjustment expediting system 300. Additionally, adjustable pressure elements 310 and common air channels 320 are closed to keep air from escaping.

In one embodiment, control circuitry 20 is arranged to measure the conductivity, or impedance, between each pair of lingual electrodes 40 and buccal electrodes 50. Particularly, an orthogonal current is generated between each lingual electrode 40 and the associated buccal electrode 50, responsive to the voltage potentials thereat. Alternately, a parallel current is generated between adjacent lingual electrodes 40 and between adjacent buccal electrodes 50. Control circuitry 20 is arranged to measure the magnitude of the generated current and optionally calculate the resistance, or conductivity, between the electrodes. The amount of pressure applied to each lingual electrode 40 and buccal electrode 50 is adjusted to arrive at the desired predetermined resistance or conductivity.

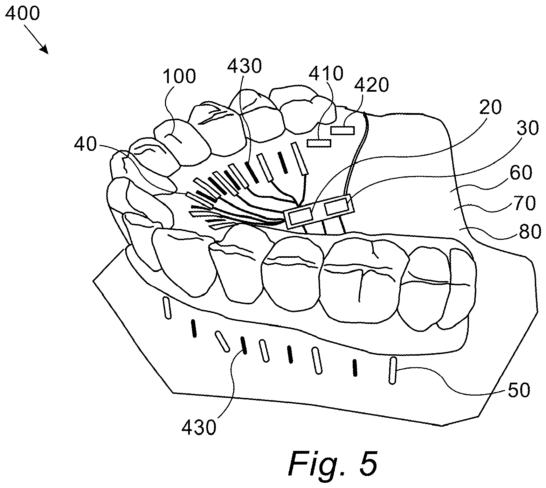

FIG. 5 illustrates a perspective view of a fourth embodiment of an orthodontic tooth adjustment expediting system 400. Orthodontic tooth adjustment expediting system 400 is in all respects similar to orthodontic tooth adjustment expediting system 10 of FIGs. 2A - 2B, with the addition of a temperature sensor 410, a pH sensor 420 and a plurality of heating elements 430, each in communication with control circuitry 20 (connections not shown). In one embodiment, each heating element 430 comprises an infrared (IR) light emitting diode (LED) 430, and is described herein as such. In one embodiment, each of the plurality of IR LEDs 430 is positioned between a respective pair of lingual electrodes 40 or buccal electrodes 50. Temperature sensor 410 and pH sensor 420 are illustrated as being positioned on gum 80 of lingual side 60 of alveolar bone 70, however this is not meant to be limiting in any way.

In operation, temperature sensor 410 is arranged to sense the temperature in the vicinity of teeth 100. Additionally, pH sensor 420 is arranged to sense the pH level in the vicinity of teeth 100. Control circuitry 20 is arranged to enable IR LEDs 430 to heat gums 80 responsive to the sensed temperature of temperature sensor 410. Particularly, control circuitry 20 is arranged to analyze the output of temperature sensor 410 and determine an average baseline temperature in the vicinity of teeth 100. IR LEDs 430 are arranged to raise the temperature 1 - 2 degrees above the determined average baseline temperature which will enhance the inflammatory response of gums 80, thereby enhancing the osteogenesis response during movement of teeth 100.

Control circuitry 20 is further arranged to control power source 30 to adjust the amplitude of the generated currents responsive to the sensed pH level. The generated currents can cause a reduction in the pH level due to an electrolytic effect around lingual electrodes 40 and buccal electrodes 50. In the event that the pH level drops below a predetermined value, the current magnitude is reduced so as to avoid excessive acidity gums 80 which can damage teeth 100.

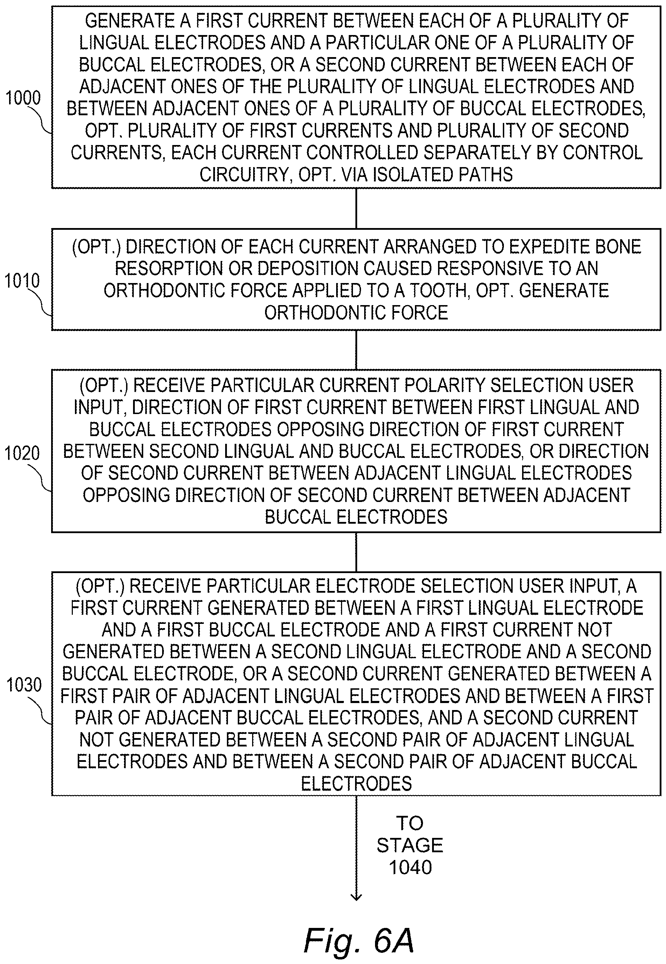

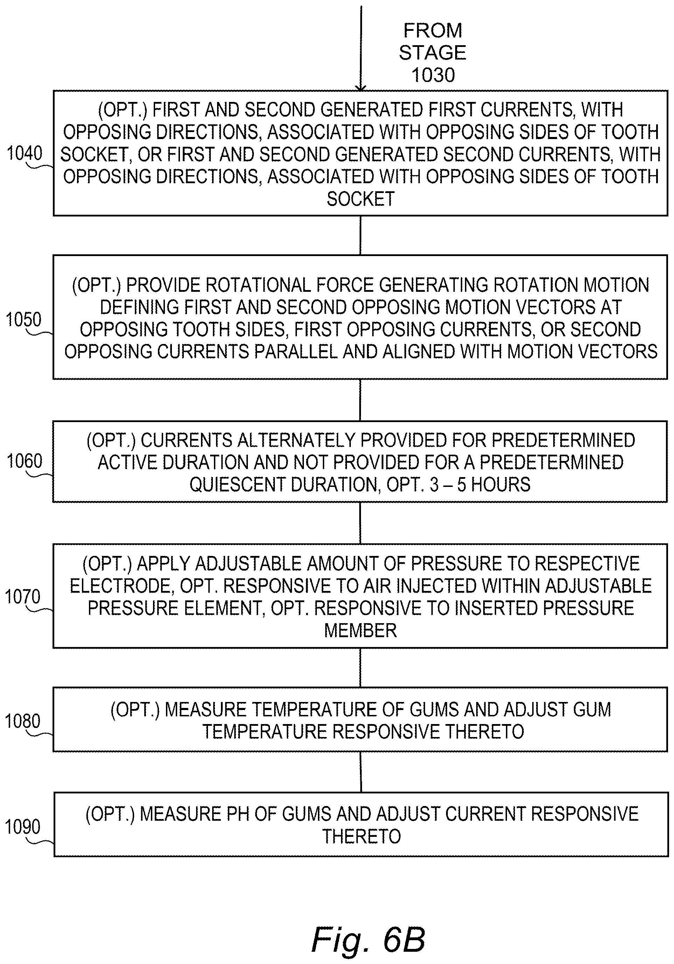

FIGs. 6A - 6B illustrate a high level flow chart of a method of expediting orthodontic tooth adjustment. In stage 1000, a first type current is generated between each of a plurality of lingual electrodes and a particular one of a plurality of buccal electrodes, as described above in relation to the generated orthogonal currents. Alternately, or additionally, a second type current is generated between each of adjacent ones of the plurality of lingual electrodes and between adjacent ones of the plurality of buccal electrodes, as described above in relation to the generated parallel currents. Optionally, a plurality of first type currents, each between a particular lingual electrode and a particular buccal electrode, and a plurality of second type currents, each between adjacent lingual electrodes or adjacent buccal electrodes, are generated. As described above, a lingual electrode is positioned between each tooth on the lingual side of the alveolar bone and a buccal electrode is positioned between each tooth on the buccal side of the alveolar bone. Each of the generated first and second type currents is controlled separately by a control circuitry. Optionally, each of the lingual and buccal electrodes is in communication with the control circuitry via a respective one of a plurality of electrical paths, isolated from the rest of the plurality of electrical paths. As will be described below, the control circuitry is arranged to control the magnitude and direction of each generated current.

In optional stage 1010, the direction of each generated current of stage 1000 is arranged so as to expedite bone resorption and/or deposition caused responsive to an orthodontic force applied to a tooth disposed within the respective tooth socket associated with the respective electrodes generating the current. Further optionally, the orthodontic force is generated by an orthodontic appliance.

In optional stage 1020, a particular current polarity selection user input is received. Responsive to the received current polarity selection user input, the direction of a generated first type current of stage 1000 between a first lingual electrode and a first buccal electrode opposes the direction of a generated first type current between a second lingual electrode and a second buccal electrode. Alternatively, or additionally, responsive to the received current polarity selection user input, the direction of a generated second type current of stage 1000 between adjacent lingual electrodes opposes the direction of a generated second type current between adjacent buccal electrodes. Particularly, the direction of each generated current is selected responsive to a user input, thereby allowing flexibility for a wide range of tooth movements, as described above.

In optional stage 1030, a particular electrode selection user input is received. Responsive to the received electrode selection user input, a first type current of stage 1000 is generated between a first lingual electrode and a first buccal electrode and another first type current is not generated between a second lingual electrode and a second buccal electrode. Alternatively, or additionally, a second type current of stage 1000 is generated between a first pair of adjacent lingual electrodes and between a first pair of adjacent buccal electrodes, and another second type current is not generated between a second pair of adjacent lingual electrodes and between a second pair of adjacent buccal electrodes. Particularly, the number of generated first and second type currents is selected responsive to a user input.

In optional stage 1040, a first and a second first type currents, exhibiting opposing directions, are generated. Each of the first type currents, i.e. orthogonal currents, is associated with an opposing side of a tooth socket. Alternatively, or additionally, a first and a second second type currents, exhibiting opposing direction, are generated. Each of the second type currents, i.e. parallel currents, is associated with an opposing side of a tooth socket. In optional stage 1050, an orthodontic rotational force is provided by an orthodontic appliance, the orthodontic rotational force generating rotational motion of a tooth. The rotational motion defines a first and a second opposing motion vectors at opposing sides of the tooth. The opposing first type currents, or second type currents, of optional stage 1040 are each generally parallel and aligned with a respective one of the motion vectors.

In optional stage 1060, the generated currents of stage 1000 are alternately provided for a predetermined active duration and not provided for a predetermined quiescent duration. Optionally, the predetermined active duration is 3 - 5 hours and the predetermined quiescent duration is 19 - 21 hours.

In optional stage 1070, an adjustable amount of pressure is applied to each of the plurality of lingual and buccal electrodes of stage 1000. Optionally, the adjustable amount of pressure is applied responsive to air being injected into a flexible tubule, the tubule expanding responsive to the injected air and applying pressure to an associated electrode. Optionally, the adjustable amount of pressure is applied responsive to a pressure member being inserter into each of the plurality of flexible tubules, the tubule expanding responsive to the inserted pressure member and applying pressure to the associated electrode.

In optional stage 1080, the temperature of the gums is sensed by a temperature sensor and the temperature of the gums is adjusted responsive to the sensed temperature. In one embodiment, the gums are heated by a pluraltiy of IR LEDs to 1 - 2 degrees above an average baseline temperature of the gums. As described above, heating the gums enhances the inflammatory response of the gums, thereby enhancing the osteogenesis response during movement of the teeth.

In optional stage 1090, the pH level of the gums is sensed by a pH sensor and the magnitude of the generated first current, or second current, of stage 1000 is adjusted responsive to the sensed pH level. Particularly, in the event that the pH level is below a predetermined value, the magnitude of the current is reduced.

Unless otherwise defined, all technical and scientific terms used herein have the same meanings as are commonly understood by one of ordinary skill in the art to which this invention belongs. Although methods similar or equivalent to those described herein can be used in the practice or testing of the present invention, suitable methods are described herein.

The terms "include", "comprise" and "have" and their conjugates as used herein mean "including but not necessarily limited to".

## Claims

1. An orthodontic tooth adjustment expediting system (200, 300, 400) comprising:
a control circuitry (20);
a power supply (30);
a lingual member (210); and
a buccal member (250), **CHARACTERIZED BY**:
said lingual member (210) being arranged to fit the contour of the gum (80) over the lingual side (60) of an alveolar bone (70);
said buccal member (250) being arranged to fit the contour of the gum (80) over the buccal side (65) of the alveolar bone (70);
a plurality of lingual electrodes (40) disposed on said lingual member (210), each of said plurality of lingual electrodes (40) adapted to be juxtaposed with the lingual side (60) of the alveolar bone (70) and associated with a particular one of a plurality of tooth sockets (90) within the alveolar bone (70), a first of said plurality of lingual electrodes (40) adapted to be juxtaposed with a first side of a first of the plurality of tooth sockets (90) and a second of said plurality of lingual electrodes (40) adapted to be juxtaposed with a second side of the first of the plurality of tooth sockets (90), the second side of the first of the plurality of tooth sockets (90) opposing the first side of the first of the plurality of tooth sockets (90); and
a plurality of buccal electrodes (50) disposed on said buccal member (250), each of said plurality of buccal electrodes (50) adapted to be juxtaposed with the buccal side (65) of the alveolar bone (70) and associated with a particular one of the plurality of tooth sockets (90) within the alveolar bone (70), a first of said plurality of buccal electrodes (50) adapted to be juxtaposed with the first side of the first of the plurality of tooth sockets (90) and a second of said plurality of buccal electrodes (50) adapted to be juxtaposed with the second side of the first of the plurality of tooth sockets (90),
wherein the system is arranged such that responsive to said control circuitry (20) and said power supply (30), an orthogonal current is generated (1040) between each of said plurality of lingual electrodes (40) and a particular one of said plurality of buccal electrodes (50), each of said generated plurality of orthogonal currents controlled separately responsive to said control circuitry (20), and
wherein the direction of said orthogonal current generated between said first lingual electrode (40) and said first buccal electrode (50) opposes the direction of said orthogonal current generated between said second lingual electrode (40) and said second buccal electrode (50).

2. The system (200, 300, 400) of claim 1, further comprising an orthodontic appliance arranged to provide (1050) a rotational force to a tooth (100) having a root within the first of the plurality of tooth sockets (90), said rotational orthodontic force arranged to generate rotational motion of the tooth (100), said generated rotational motion defining:
a first motion vector in a first direction at a portion of a first side (150) of the tooth (100); and
a second motion vector, in a second direction opposing said first direction, at a portion of a second side (170) of the tooth (100), the second side (170) of the tooth (100) opposing the first side (150) of the tooth (100),
wherein a first axis (120) extending between said first of said plurality of lingual electrodes (40) and said first of said plurality of buccal electrodes (50) is parallel and aligned with said first motion vector,
wherein a second axis (130) extending between said second of said plurality of lingual electrodes (40) and said second of said plurality of buccal electrodes (50) is parallel and aligned with said second motion vector, and
wherein the direction of said generated first orthogonal current and the direction of said generated second orthogonal current are arranged to expedite bone resorption and deposition caused responsive to said provided (1050) rotational orthodontic force.

3. The system (200, 300, 400) of claim 1, wherein each of said plurality of lingual electrodes (40) and each of said plurality of buccal electrodes (50) extends from 1 millimeter apical to the gingival margin to the mucogingival junction of the gum (80).

4. The system (200, 300, 400) of claim 1, wherein each of said plurality of lingual electrodes (40) and each of said plurality of buccal electrodes (50) extends from 1 millimeter apical to the gingival margin to between 1 - 2 millimeters over the mucosal tissue of the gum (80).

5. The system (200, 300, 400) of any of claims 1 - 4, wherein the system is arranged such that responsive to said control circuitry and said power supply, a plurality of parallel currents are generated between each of adjacent ones of said plurality of lingual electrodes and between adjacent ones of said plurality of buccal electrodes.

6. The system (200, 300, 400) of claim 1, further comprising a plurality of electrical paths (35) isolated from each other, said control circuitry (20) in electrical communication with each of said plurality of lingual electrodes (40), or with each of said plurality of buccal electrodes (50), via a respective one of said plurality of electrical paths (35).

7. The system (200, 300, 400) of claim 1, wherein said control circuitry (20) comprises a user input module (190) arranged to receive (1020) a current polarity selection user input,
wherein the directions of said generated first and second orthogonal currents are responsive to a particular current polarity selection user input received at said user input module (190).

8. The system (200, 300, 400) of claim 1, wherein said control circuitry (20) comprises a user input module (190) arranged to receive (1030) an electrode selection user input,
wherein the system is arranged such that responsive to a particular electrode selection user input received at said user input module (190), an orthogonal current is generated between a third of said plurality of lingual electrodes and a third of said plurality of buccal electrodes and an orthogonal current is not generated (1030) between a fourth of said plurality of lingual electrodes and a fourth of said plurality of buccal electrodes.

9. The system (200, 300, 400) of any of claims 1 - 4, wherein, for each of said generated orthogonal currents, said control circuitry (20) is arranged (1060) such that said generated orthogonal current is alternatively provided for a predetermined active duration and not provided for a predetermined quiescent duration, said predetermined active duration being 3 - 5 hours.

10. The system (300) of claim 1, further comprising a plurality of adjustable pressure elements (310), each of said adjustable pressure elements (310) arranged to apply (1070) an adjustable amount of pressure to a respective one of said plurality of lingual electrodes (40) and said plurality of buccal electrodes (50).

11. The system (300) of claim 10, wherein each of said adjustable pressure elements (310) is arranged (1070) to expand responsive to air being injected therewithin, said adjustable amount of pressure applied responsive to said injected air.

12. The system of claim 10, wherein each of said adjustable pressure elements (310) is arranged (1070) to expand responsive to a pressure member being inserted therewithin, said adjustable amount of pressure applied responsive to said inserted pressure member.

13. The system (400) of claim 1, further comprising:
a temperature sensor (410) arranged to sense (1080) the temperature of the gums (80) of the alveolar bone (70); and
a heating element (430),
wherein said control circuitry (20) is arranged to analyze an output of said temperature sensor (410) and determine an average baseline temperature in the vicinity of teeth (100) having roots within the plurality of tooth sockets (90), and
wherein said heating element (430) is arranged to heat (1080) the gums (80) of the alveolar bone (70) 1-2 degrees above said determined average baseline temperature.

14. The system (400) of claim 1, further comprising a pH sensor (420) arranged to sense (1090) the pH level of the gums (80) of the alveolar bone (70),
wherein the magnitude of said generated orthogonal currents are adjusted (1090) responsive to said sensed pH level.

## Patentansprüche

1. Kieferorthopädisches System zur beschleunigten Zahnanpassung (200, 300, 400), das Folgendes beinhaltet:
eine Steuerschaltung (20);
eine Stromversorgung (30);
ein linguales Element (210); und
ein bukkales Element (250), **DADURCH GEKENNZEICHNET, DASS**:
das linguale Element (210) angeordnet ist, um die Kontur des Zahnfleisches (80) über der lingualen Seite (60) eines Alveolarknochens (70) anzupassen;
das bukkale Element (250) angeordnet ist, um die Kontur des Zahnfleisches (80) über der bukkalen Seite (65) des Alveolarknochens (70) anzupassen;
eine Vielzahl von Lingualelektroden (40) auf dem lingualen Element (210) angeordnet ist, wobei jede der Vielzahl von Lingualelektroden (40) dazu ausgelegt ist, um auf der lingualen Seite (60) des Alveolarknochens (70) nebeneinander angeordnet und einer bestimmten aus einer Vielzahl von Zahnfächern (90) innerhalb des Alveolarknochens (70) zugeordnet zu werden, wobei eine erste der Vielzahl von Lingualelektroden (40) dazu ausgelegt ist, um auf einer ersten Seite einer ersten der Vielzahl von Zahnfächern (90) nebeneinander angeordnet zu werden, und eine zweite der Vielzahl von Lingualelektroden (40) dazu ausgelegt ist, um auf einer zweiten Seite der ersten der Vielzahl von Zahnfächern (90) nebeneinander angeordnet zu werden, wobei die zweite Seite der ersten der Vielzahl von Zahnfächern (90) gegenüber der ersten Seite der ersten der Vielzahl von Zahnfächern (90) liegt; und
eine Vielzahl von Bukkalelektroden (50) auf dem bukkalen Element (250) angeordnet ist, wobei jede der Vielzahl von Bukkalelektroden (50) ausgelegt ist, um auf der bukkalen Seite (65) des Alveolarknochens (70) nebeneinander angeordnet und einer bestimmten aus der Vielzahl von Zahnfächern (90) innerhalb des Alveolarknochens (70) zugeordnet zu werden, wobei eine erste aus der Vielzahl von Bukkalelektroden (50) dazu ausgelegt ist, um auf der ersten Seite der ersten der Vielzahl von Zahnfächern (90) nebeneinander angeordnet zu werden, und wobei eine zweite aus der Vielzahl von Bukkalelektroden (50) dazu ausgelegt ist, um auf der zweiten Seite der ersten aus der Vielzahl von Zahnfächern (90) nebeneinander angeordnet zu werden,
wobei das System so angeordnet ist, dass,
auf die Steuerschaltung (20) und die Stromversorgung (30) ansprechend,
ein orthogonaler Strom (1040) zwischen jeder der Vielzahl von Lingualelektroden (40) und einer bestimmten der Vielzahl von Bukkalelektroden (50) erzeugt wird,
wobei jeder der erzeugten Vielzahl von orthogonalen Strömen, die separat gesteuert werden, auf die Steuerschaltung (20) anspricht, und
wobei die Richtung des zwischen der ersten Lingualelektrode (40) und der ersten Bukkalelektrode (50) erzeugten orthogonalen Stroms der Richtung des zwischen der zweiten Lingualelektrode (40) und der zweiten Bukkalelektrode (50) erzeugten orthogonalen Stroms entgegengesetzt ist.

2. System (200, 300, 400) nach Anspruch 1, das ferner eine kieferorthopädische Vorrichtung umfasst, die konstruiert ist, um eine Rotationskraft auf einen Zahn (100) mit einer Wurzel innerhalb der ersten der Vielzahl von Zahnfächern (90) auszuüben (1050), wobei die kieferorthopädische Rotationskraft derart beschaffen ist, dass sie eine Drehbewegung des Zahnes (100) erzeugt, wobei die erzeugte Drehbewegung einen ersten Bewegungsvektor in einer ersten Richtung an einem Abschnitt einer ersten Seite (150) des Zahnes (100) definiert; und einen zweiten Bewegungsvektor in einer der ersten Richtung entgegengesetzten zweiten Richtung an einem Abschnitt einer zweiten Seite (170) des Zahnes (100) definiert, wobei die zweite Seite (170) des Zahnes (100) der ersten Seite (150) des Zahnes (100) gegenüberliegt, wobei eine erste Achse (120), die zwischen der ersten der Vielzahl von Lingualelektroden (40) und der ersten der Vielzahl von Bukkalelektroden (50) verläuft, parallel ist und mit dem ersten Bewegungsvektor ausgerichtet ist,
wobei eine zweite Achse (130), die zwischen der zweiten der Vielzahl von Lingualelektroden (40) und der zweiten der Vielzahl von Bukkalelektroden (50) verläuft, parallel ist und mit dem zweiten Bewegungsvektor ausgerichtet ist, und
wobei die Richtung des erzeugten ersten orthogonalen Stroms und die Richtung des erzeugten zweiten orthogonalen Stroms angeordnet sind, um die Knochenresorption und -ablagerung zu beschleunigen, die als Reaktion auf die ausgeübte (1050) orthodontische Rotationskraft verursacht wird.

3. System (200, 300, 400) nach Anspruch 1, wobei sich jede der Vielzahl von Lingualelektroden (40) und jede der Vielzahl von Bukkalelektroden (50) von 1 Millimeter apikal über den Zahnfleischrand bis hin zur Mukogingivalgrenze des Zahnfleisches (80) erstreckt.

4. System (200, 300, 400) nach Anspruch 1, wobei sich jede der Vielzahl von Lingualelektroden (40) und jede der Vielzahl von Bukkalelektroden (50) von 1 Millimeter apikal zum Zahnfleischrand bis hin zu 1 - 2 Millimetern über das Schleimhautgewebe des Zahnfleisches (80) erstreckt.

5. System (200, 300, 400) nach einem der Ansprüche 1 - 4, wobei das System so angeordnet ist, dass, auf die Steuerschaltung und die Stromversorgung ansprechend, eine Vielzahl von parallelen Strömen zwischen jeder der benachbarten aus der Vielzahl von Lingualelektroden und zwischen benachbarten aus der Vielzahl von Bukkalelektroden erzeugt wird.

6. System (200, 300, 400) nach Anspruch 1, das ferner eine Vielzahl von voneinander isolierten elektrischen Leitern (35) umfasst, wobei die Steuerschaltung (20) in elektrischer Verbindung mit jeder der Vielzahl von Lingualelektroden (40) oder mit jeder der Vielzahl von Bukkalelektroden (50) über einen entsprechenden elektrischen Leiter aus der Vielzahl von elektrischen Leitern (35) steht.

7. System (200, 300, 400) nach Anspruch 1, wobei die Steuerschaltung (20) ein Benutzereingabemodul (190) enthält, das zum Empfangen (1020) einer Benutzereingabe zur Auswahl der Strompolarität angeordnet ist,
wobei die Richtungen des erzeugten ersten und zweiten orthogonalen Stroms auf eine bestimmte Benutzereingabe zur Auswahl der Strompolarität reagieren, die am Benutzereingabemodul (190) empfangen wird.

8. System (200, 300, 400) nach Anspruch 1, wobei die Steuerschaltung (20) ein Benutzereingabemodul (190) enthält, das zum Empfangen (1030) einer Benutzereingabe zur Elektrodenauswahl angeordnet ist, wobei das System so angeordnet ist, dass als Reaktion auf eine bestimmte Elektrodenauswahl-Benutzereingabe, die am Benutzereingabemodul (190) empfangen wird, ein orthogonaler Strom zwischen einer dritten aus der Vielzahl von Lingualelektroden und einer dritten aus der Vielzahl von Bukkalelektroden erzeugt wird und kein orthogonaler Strom (1030) zwischen einer vierten aus der Vielzahl von Lingualelektroden und einer vierten aus der Vielzahl von Bukkalelektroden erzeugt wird.

9. System (200, 300, 400) nach einem der Ansprüche 1- 4, wobei für jeden der erzeugten orthogonalen Ströme die Steuerschaltung (20) so angeordnet ist (1060), dass der erzeugte orthogonale Strom alternativ für eine vorbestimmte aktive Dauer und nicht für eine vorbestimmte Ruhezeit vorgesehen ist, wobei die vorbestimmte aktive Dauer 3 - 5 Stunden beträgt.

10. System (300) nach Anspruch 1, das ferner eine Vielzahl von einstellbaren Druckelementen (310) enthält, wobei jedes der einstellbaren Druckelemente (310) angeordnet ist, um eine einstellbare Druckmenge auf eine jeweilige der Vielzahl von Lingualelektroden (40) sowie der Vielzahl von Bukkalelektroden (50) auszuüben (1070).

11. System (300) nach Anspruch 10, wobei jedes der einstellbaren Druckelemente (310) so angeordnet ist (1070), dass es sich als Reaktion auf die darin eingespritzte Luft ausdehnt, wobei die regelbare Druckmenge als Reaktion auf die eingespritzte Luft angewendet wird.

12. System nach Anspruch 10, wobei jedes der einstellbaren Druckelemente (310) so angeordnet ist (1070), dass es sich als Reaktion auf ein darin eingesetztes Druckelement ausdehnt, wobei die regelbare Druckmenge als Reaktion auf das eingesetzte Druckelement angewendet wird.

13. System (400) nach Anspruch 1, das ferner Folgendes umfasst:
einen Temperatursensor (410), der angeordnet ist, um die Temperatur des Zahnfleisches (80) am Alveolarknochen (70) zu erfassen (1080); sowie ein Heizelement (430),
wobei die Steuerschaltung (20) angeordnet ist, um ein Ausgangssignal des Temperatursensors (410) zu analysieren und eine durchschnittliche Ausgangstemperatur in der Nähe von Zähnen (100) mit Wurzeln innerhalb der Vielzahl von Zahnfächern (90) zu bestimmen, und
wobei das Heizelement (430) konstruiert ist, um das Zahnfleisch (80) am Alveolarknochen (70) auf 1-2 Grad über der bestimmten durchschnittlichen Ausgangstemperatur zu erwärmen (1080).

14. System (400) nach Anspruch 1, das ferner einen pH-Sensor (420) enthält, der angeordnet ist, um den pH-Wert des Zahnfleisches (80) am Alveolarknochen (70) zu messen (1090),
wobei die Größe der erzeugten orthogonalen Ströme als Reaktion auf den gemessenen pH-Wert angepasst (1090) wird.

## Revendications

1. Système d'accélération du réglage dentaire orthodontique (200, 300, 400), comprenant :
un circuit de commande 20 ;
une alimentation électrique (30) ;
un élément lingual (210) ; et
un élément buccal (250), **CARACTÉRISÉ EN CE QUE** :
ledit élément lingual (210) étant agencé pour s'ajuster au contour de la gencive (80) sur le côté lingual (60) d'un os alvéolaire (70) ;
ledit élément buccal (250) étant agencé pour s'ajuster au contour de la gencive (80) sur le côté buccal (65) de l'os alvéolaire (70) ;
une pluralité d'électrodes linguales (40) disposées sur ledit élément lingual (210), chacune de ladite pluralité d'électrodes linguales (40) étant adaptée pour être juxtaposée au côté lingual (60) de l'os alvéolaire (70) et associée à une alvéole dentaire particulière d'une pluralité d'alvéoles dentaires (90) dans l'os alvéolaire (70), une première de ladite pluralité d'électrodes linguales (40) adaptée pour être juxtaposée à un premier côté d'une première face d'une première de la pluralité d'alvéoles dentaires (90) et une seconde de ladite pluralité d'électrodes linguales (40) adaptée pour être juxtaposée à un second côté de la première de la pluralité d'alvéoles dentaires (90), le second côté de la première de la pluralité d'alvéoles dentaires (90) étant opposé au premier côté de la première de la pluralité d'alvéoles dentaires (90) ; et
une pluralité d'électrodes buccales (50) disposées sur ledit élément buccal (250), chacune de ladite pluralité d'électrodes buccales (50) étant adaptée pour être juxtaposée au côté buccal (65) de l'os alvéolaire (70) et associée à une alvéole particulière de la pluralité d'alvéoles dentaires (90) dans l'os alvéolaire (70), une première de ladite pluralité d'électrodes buccales (50) adaptée pour être juxtaposée au premier côté du premier de la pluralité d'alvéoles dentaires (90) et une seconde de ladite pluralité d'électrodes buccales (50) adaptée pour être juxtaposée au second côté de la première de la pluralité d'alvéoles dentaires (90), le système étant agencé de sorte qu'en réaction audit circuit de commande (20) et à ladite alimentation électrique (30), un courant orthogonal soit généré (1040) entre chacune de ladite pluralité d'électrodes linguales (40) et une électrode particulière de ladite pluralité d'électrodes buccales (50), chacune de ladite pluralité générée de courants orthogonaux commandés séparément en réaction audit circuit de commande (20), et la direction dudit courant orthogonal généré entre ladite première électrode linguale (40) et ladite première électrode buccale (50) soit opposée à la direction dudit courant orthogonal généré entre ladite seconde électrode linguale (40) et ladite seconde électrode buccale (50).

2. Système (200, 300, 400) selon la revendication 1, comprenant en outre un appareil orthodontique agencé pour fournir (1050) une force de rotation à une dent (100) ayant une racine dans la première de la pluralité d'alvéoles dentaires (90), ladite force orthodontique de rotation étant agencée pour générer un mouvement de rotation de la dent (100), ledit mouvement de rotation généré définissant :
un premier vecteur de mouvement dans une première direction à une partie d'un premier côté (150) de la dent (100) ; et
un second vecteur de mouvement, dans une seconde direction opposée à ladite première direction, sur une partie d'un second côté (170) de la dent (100), le second côté (170) de la dent (100) étant opposée au premier côté (150) de la dent (100),
un premier axe (120) s'étendant entre ladite première de ladite pluralité d'électrodes linguales (40) et ladite première de ladite pluralité d'électrodes buccales (50) étant parallèle et aligné sur ledit premier vecteur de mouvement,
un second axe (130) s'étendant entre ladite seconde de ladite pluralité d'électrodes linguales (40) et ladite seconde de ladite pluralité d'électrodes buccales (50) étant parallèle et aligné sur ledit second vecteur de mouvement, et
la direction dudit premier courant orthogonal généré et la direction dudit second courant orthogonal généré étant agencés pour accélérer la résorption et le dépôt osseux provoqués en réaction à ladite force orthodontique de rotation fournie (1050).

3. Système (200, 300, 400) selon la revendication 1, dans lequel chacune de ladite pluralité d'électrodes linguales (40) et chacune de ladite pluralité d'électrodes buccales (50) s'étend de 1 millimètre apical par rapport au bord gingival de la jonction mucogingivale de la gencive (80).

4. Système (200, 300, 400) selon la revendication 1, dans lequel chacune de ladite pluralité d'électrodes linguales (40) et chacune de ladite pluralité d'électrodes buccales (50) s'étend de 1 millimètre apical par rapport au bord gingival jusqu'à 1 à 2 millimètres sur le tissu muqueux de la gencive (80).

5. Système (200, 300, 400) selon l'une quelconque des revendications 1 à 4, dans lequel le système est agencé, de sorte qu'en réaction audit circuit de commande et à ladite alimentation électrique, une pluralité de courants parallèles soient générés entre chacune des électrodes adjacentes de ladite pluralité d'électrodes linguales et entre des électrodes adjacentes de ladite pluralité d'électrodes buccales.

6. Système (200, 300, 400) selon la revendication 1, comprenant en outre une pluralité de chemins électriques (35) isolés les uns des autres, ledit circuit de commande (20) en communication électrique avec chacune de ladite pluralité d'électrodes linguales (40), ou avec chacune de ladite pluralité d'électrodes buccales (50), par l'intermédiaire d'un chemin respectif de ladite pluralité de chemins électriques (35).

7. Système (200, 300, 400) selon la revendication 1, dans lequel ledit circuit de commande (20) comprend un module d'entrée utilisateur (190) agencé pour recevoir (1020) une entrée utilisateur de sélection de polarité de courant,
dans lequel les directions desdits premier et second courants orthogonaux générés sont en réaction à une entrée utilisateur de sélection de polarité de courant particulière reçue au niveau dudit module d'entrée utilisateur (190).

8. Système (200, 300, 400) selon la revendication 1, dans lequel ledit circuit de commande (20) comprend un module d'entrée utilisateur (190) agencé pour recevoir (1030) une entrée utilisateur de sélection d'électrode,
le système étant agencé, de sorte qu'en réaction à une entrée utilisateur de sélection d'électrode particulière reçue au niveau dudit module d'entrée utilisateur (190), un courant orthogonal soit généré entre une troisième de ladite pluralité d'électrodes linguales et une troisième de ladite pluralité d'électrodes buccales et qu'un courant orthogonal ne soit pas généré (1030) entre une quatrième de ladite pluralité d'électrodes linguales et une quatrième de ladite pluralité d'électrodes buccales.

9. Système (200, 300, 400) selon l'une quelconque des revendications 1 à 4, dans lequel, pour chacun desdits courants orthogonaux générés, ledit circuit de commande (20) est agencé (1060) de sorte que ledit courant orthogonal généré soit alternativement fourni pendant une durée active prédéterminée et non fourni pendant une durée de repos prédéterminée, ladite durée active prédéterminée étant de 3 à 5 heures.

10. Système (300) selon la revendication 1, comprenant en outre une pluralité d'éléments de pression réglables (310), chacun desdits éléments de pression réglables (310) étant agencé pour appliquer (1070) une quantité réglable de pression à une électrode respective de ladite pluralité d'électrodes linguales (40) et de ladite pluralité d'électrodes buccales (50).

11. Système (300) selon la revendication 10, dans lequel chacun desdits éléments de pression réglables (310) est agencé (1070) pour se dilater en réaction à l'injection de l'air dans celui-ci, ladite quantité réglable de pression étant appliquée en réaction audit air injecté.

12. Système selon la revendication 10, dans lequel chacun desdits éléments de pression réglables (310) est agencé (1070) pour se dilater en réaction à l'insertion d'un élément de pression dans celui-ci, ladite quantité de pression réglable étant appliquée en réaction audit élément de pression inséré.

13. Système (400) selon la revendication 1, comprenant en outre :
un capteur de température (410) agencé pour détecter (1080) la température des gencives (80) de l'os alvéolaire (70) ; et
un élément chauffant (430),
dans lequel ledit circuit de commande (20) est agencé pour analyser une sortie dudit capteur de température (410) et déterminer une température de base moyenne dans le voisinage des dents (100) ayant des racines dans la pluralité de logements de dents (90), et
dans lequel ledit élément chauffant (430) est agencé pour chauffer (1080) les gencives (80) de l'os alvéolaire (70) de 1 à 2 degrés au-dessus de ladite température moyenne déterminée.

14. Système (400) selon la revendication 1, comprenant en outre un capteur de pH (420) agencé pour détecter (1090) le niveau de pH des gencives (80) de l'os alvéolaire (70),
l'amplitude desdits courants orthogonaux générés étant ajustée (1090) en réaction audit niveau de pH détecté.
